# EUROPEAN PATENT SPECIFICATION

(11) **EP 3 630 787 B1**
(45) Date of publication and mention of the grant of the patent: **15.01.2025**
(21) Application number: 18728386.6
(22) Date of filing: 01.06.2018
(51) Int. Cl.: C07H 21/00, B82Y 5/00, A61K 31/713, C12N 15/11

(54) **TETRAMOLECULAR PARALLEL G-QUADRUPLEX-FORMING HYDROPHOBICALLY MODIFIED OLIGONUCLEOTIDES**
TETRAMOLEKULARE PARALLELE G-QUADRUPLEX-BILDENDE HYDROPHOB-MODIFIZIERTE OLIGONUKLEOTIDE
OLIGONUCLÉOTIDES MODIFIÉS DE MANIÈRE HYDROPHOBE FORMANT DES G-QUADRUPLEXES PARALLÈLES TÉTRAMOLÉCULAIRES

(30) Priority: 02.06.2017 EP 17305651
(43) Date of publication of application: 08.04.2020
(73) Proprietor: Institut National de la Santé et de la Recherche Médicale (INSERM), 75013 Paris (FR); Centre National de la Recherche Scientifique, 75016 Paris (FR); Université de Bordeaux, 33000 Bordeaux (FR)
(72) Inventor: BARTHELEMY, Philippe, 33700 Merignac (FR); GISSOT, Arnaud, 33076 Bordeaux Cedex (FR); VIALET, Brune, 33076 Bordeaux (FR)
(74) Representative: Lavoix
(86) International application number: PCT/EP2018/064416
(87) International publication number: WO 2018/220154

(56) References cited:
- EP-A1- 3 147 364
- WO-A1-2014/195430
- WO-A1-2014/195432
- WO-A1-2015/153975

## Description

The present invention concerns tetramolecular parallel G-quadruplex-forming oligonucleotides.

Guanine-rich oligonucleotide sequences can self-assemble into four-stranded G-quadruplex supramolecular structures. These structures, which are stabilized by π-π stacking between G-quartets (G4) and via Hoogsteen hydrogen bonding have been found for instance in telomeric and promoter regions, where they participate in a diversity of biological processes. Due to their unique biological and biophysical properties, G-quadruplex supramolecular structures have been intensively investigated in different areas including supramolecular chemistry, nanotechnology or medicinal chemistry.

G-quadruplexes can fold with different strand stoichiometries (one to four strands), orientations (parallel, antiparallel, hybrid), and different number of G-quartets.

If G-quadruplexes are of prime importance in biology, their acceptance as useful and versatile supramolecular scaffolds is still hampered by the propensity of G4-prone DNA or RNA molecules to adopt many different G4 topological conformations (also called polymorphism), or other alternative foldings.

Obviously, the probability of forming undesired foldings from G4-prone oligonucleotide (ON) sequences increases with the ON length. Besides the above mentioned issues, tetramolecular G-quadruplexes structures exhibit very slow kinetics of formation due to the unfavorable entropy associated with the formation of a quaternary complex. Accordingly, it is not surprising that only short-size tetramolecular G-quadruplexes have been described so far.

If these shortcomings (kinetics and/or conformational diversity) were alleviated, tetramolecular G-quadruplexes would appear as attractive supramolecular scaffolds as they allow for the assembly of four strands in a perfectly controlled, predictable and unique tetramolecular architecture.

EP3147364 A1 discloses oligonucleotides modified by substitution at the 5' or the 3' end by a lipid moiety, wherein said oligonucleotide comprises a nucleic acid sequence of at least 10 nucleotides, said nucleic acid sequence including a series of at least 4 consecutive guanine residues located in the middle of said sequence.

One obvious workaround consists in the synthesis of a spatially pre-organized complex where the four strands are covalently attached to a cyclic template. Kinetically speaking, this turns a high order quaternary complex into a more favorable first order process. Although very elegant, this strategy is of limited practical utility as it requires extensive synthetic work.

There is thus still an important need of tetramolecular G-quadruplexes with sufficiently rapid kinetics of formation and a low level of undesired foldings.

The present invention arises from the unexpected finding by the inventors that lipid modification of ON is very useful for the folding of otherwise unfavorable long tetramolecular parallel G-quadruplexes (tpG4).

Hybrid Lipid-OligoNucleotides (LONs) are emerging as a new class of synthetic biomolecules, suitable for therapeutic and technological applications. Their amphiphilic nature impart them with self-assembling properties and LONs were found to come in a diversity of different supramolecular structures depending on the length of the oligonucleotide sequence, the nature of the linker etc.

However, the possibility of forming long tpGA using LONs bearing a long DNA had not been disclosed nor suggested.

The present invention thus concerns an oligonucleotide modified by substitution at the 5' or the 3' end by a lipid moiety, wherein said oligonucleotide comprises a nucleic acid sequence of at least 10 nucleotides, said DNA sequence including a series of at least 4 consecutive guanine residues located in the middle of said sequence.

The present invention also concerns a tetramolecular parallel G-quadruplex comprising 4 identical modified oligonucleotides as defined above, wherein each of the 4 consecutive guanine residues included in the middle of the nucleic acid sequence of each oligonucleotide respectively forms G-quartets with the corresponding guanine residues of the other 3 oligonucleotides, said G-quartets being stabilized by π-π staking and Hoogsteen hydrogen bonding.

Another object of the invention concerns a composition comprising tetramolecular parallel G-quadruplexes as defined above, wherein the tetramolecular parallel G-quadruplexes self-assembled into micelles.

The present invention further concerns the use of the composition as defined above as a vehicle.

The present invention also concerns the composition as defined above for use as a medicament.

Another object of the invention concerns the use of a tetramolecular parallel G-quadruplex as defined above in the manufacture of nanotechnology devices.

Another object of the invention relates to the tetramolecular parallel G-quadruplex as defined above for use as artificial implant.

### Detailed description of the invention

### Oligonucleotide

As used herein, the term "oligonucleotide" refers to a nucleic acid sequence which may be 3'-5' or 5'-3' oriented. The oligonucleotide used in the context of the invention may in particular be DNA or RNA. In a particular embodiment, the oligonucleotide used in the context of the invention is DNA.

The oligonucleotide used in the context of the invention comprises or consists of a nucleic acid sequence, in particular a DNA sequence, of at least 10 nucleotides, preferably at least 11 nucleotides, at least 12 nucleotides, at least 13 nucleotides, at least 14 nucleotides or at least 15 nucleotides. In particular, the oligonucleotide used in the context of the invention comprises a nucleic acid sequence, in particular a DNA sequence, of 10 nucleotides to 100 kb. Preferably, the oligonucleotide used in the context of the invention comprises or consists of a nucleic acid sequence, in particular a DNA sequence, of at least 16 nucleotides, at least 17 nucleotides, at least 18 nucleotides, at least 19 nucleotides or at least 20 nucleotides. In a particular embodiment, the oligonucleotide used in the context of the invention comprises or consists of a nucleic acid sequence, in particular a DNA sequence, of 19 nucleotides.

In the context of the invention, said nucleic acid sequence, in particular said DNA sequence, includes a series of at least 4 consecutive guanine residues located in the middle of said sequence.

In the context of the invention, the number of nucleotides forming the nucleic acid sequence defined above include the at least 4 consecutive guanine residues defined above.

By "located in the middle of the sequence" is meant herein that the series of guanine residues is not located at the 5' end or at the 3' end of the nucleic acid sequence, and is separated from each end of the nucleic acid sequence by a number of nucleotides corresponding to at least 25%, preferably at least 30%, at least 35%, at least 40% or at least 45% of the total number of nucleotides of the nucleic acid sequence, the number of nucleotides separating the series of guanine from the 5' end and the 3' end of the nucleic acid sequence being identical or different.

The oligonucleotides used in the context of the invention may be further modified, preferably chemically modified, in order to increase the stability of the oligonucleotides *in vivo.* In particular, the oligonucleotide used in the context of the invention may comprise modified nucleotides.

Chemical modifications may occur at three different sites: (i) at phosphate groups, (ii) on the sugar moiety, and/or (iii) on the entire backbone structure of the oligonucleotide.

For example, the oligonucleotides may be employed as phosphorothioate derivatives (replacement of a non-bridging phosphoryl oxygen atom with a sulfur atom) which have increased resistance to nuclease digestion. 2'-methoxyethyl (MOE) modification (such as the modified backbone commercialized by ISIS Pharmaceuticals) is also effective.

Additionally or alternatively, the oligonucleotides of the invention may comprise completely, partially or in combination, modified nucleotides which are derivatives with substitutions at the 2' position of the sugar, in particular with the following chemical modifications: O-methyl group (2'-O-Me) substitution, 2-methoxyethyl group (2'-O-MOE) substitution, fluoro group (2'-fluoro) substitution, chloro group (2'-CI) substitution, bromo group (2'-Br) substitution, cyanide group (2'-CN) substitution, trifluoromethyl group (2'-CF₃) substitution, OCF₃ group (2'-OCF₃) substitution, OCN group (2'-OCN) substitution, O-alkyl group (2'-O-alkyl) substitution, S-alkyl group (2'-S-alkyl) substitution, N-alkyl group (2'-N-akyl) substitution, O-alkenyl group (2'-O-alkenyl) substitution, S-alkenyl group (2'-S-alkenyl) substitution, N-alkenyl group (2'-N-alkenyl) substitution, SOCH₃ group (2'-SOCH₃) substitution, SO₂CH₃ group (2'-SO₂CH₃) substitution, ONO₂ group (2'-ONO₂) substitution, NO₂ group (2'-NO₂) substitution, N₃ group (2'-N₃) substitution and/or NH₂ group (2'-NH₂) substitution.

Additionally or alternatively, the oligonucleotides of the invention may comprise completely or partially modified nucleotides wherein the ribose moiety is used to produce locked nucleic acid (LNA), in which a covalent bridge is formed between the 2' oxygen and the 4' carbon of the ribose, fixing it in the 3'-endo configuration. These constructs are extremely stable in biological medium, able to activate RNase H and form tight hybrids with complementary RNA and DNA.

Accordingly, in a preferred embodiment, the oligonucleotide used in the context of the invention comprises modified nucleotides selected from the group consisting of LNA, 2'-OMe analogs, 2'-phosphorothioate analogs, 2'-fluoro analogs, 2'-CI analogs, 2'-Br analogs, 2'-CN analogs, 2'-CF₃ analogs, 2'-OCF₃ analogs, 2'-OCN analogs, 2'-O-alkyl analogs, 2'-S-alkyl analogs, 2'-N-alkyl analogs, 2'-O-alkenyl analogs, 2'-S-alkenyl analogs, 2'-N-alkenyl analogs, 2'-SOCH₃ analogs, 2'-SO₂CH₃ analogs, 2'-ONO₂ analogs, 2'-NO₂ analogs, 2'-N₃ analogs, 2'-NH₂ analogs and combinations thereof. More preferably, the modified nucleotides are selected from the group consisting of LNA, 2'-OMe analogs, 2'-phosphorothioate analogs and 2'-fluoro analogs.

Additionally or alternatively, some nucleobases of the oligonucleotide may be present as desoxyriboses. That modification should only affect the skeleton of the nucleobase, in which the hydroxyl group is absent, but not the side chain of the nucleobase which remains unchanged.

In a preferred embodiment, the guanines of the series of at least 4 consecutive guanines included in the nucleic acid sequence, as defined above, are not modified nucleotides.

In a particularly preferred embodiment, the oligonucleotide used in the context of the invention comprises or consists of the sequence 5'-TTAGTTGGGGTTCAGTTGG-3' (SEQ ID NO: 1).

### Lipid conjugate

The present invention concerns an oligonucleotide modified by substitution at the 5' or the 3' end by a lipid moiety, wherein said oligonucleotide comprises a nucleic acid sequence of at least 10 nucleotides, said nucleic acid sequence including a series of at least 4 consecutive guanine residues located in the middle of said sequence.

In the context of the invention, the term "alkyl" refers to a hydrocarbon chain that may be a linear or branched chain, containing the indicated number of carbon atoms. For example, C₁-C₁₂ alkyl indicates that the group may have from 1 to 12 (inclusive) carbon atoms in it.

In the context of the invention, the term "acyl" refers to an alkylcarbonyl, cycloalkylcarbonyl, arylcarbonyl, heterocyclylcarbonyl or heteroarylcarbonyl substituent.

Preferably, the oligonucleotide sequence "Oligo-" is connected to the divalent linker moiety X via a phosphate moiety -O-P(=O)(O⁻)-, at its 3' or 5' end, advantageously at its 5' end.

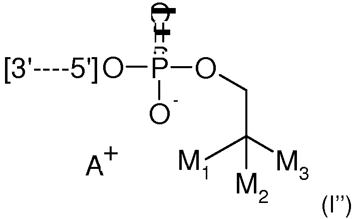

The modified oligonucleotide of the invention is of the general formula (II) 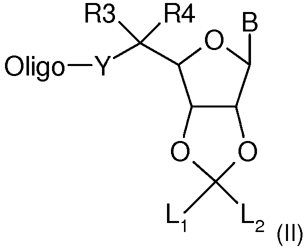 wherein:
- Oligo represents a nucleic acid sequence, preferably a DNA sequence, of at least 10 nucleotides, in particular of at least 15 nucleotides, said nucleic acid sequence including a series of at least 4 consecutive guanine residues located in the middle of said sequence, wherein said nucleic acid sequence may be oriented 3'-5' or 5'-3' and/or may comprise modified nucleotides;
- Y represents a divalent linker moiety selected from ether -O-, thio - S-, amino -NH-, and methylene -CH₂-;
- R₃ and R₄ may be identical or different and represent:
   ∘ a hydrogen atom,
   ∘ a halogen atom, in particular fluorine atom,
   ∘ a hydroxyl group, or
   ∘ an alkyl group comprising from 1 to 12 carbon atoms;
- L₁ and L₂ may be identical or different and represent a saturated or unsaturated, linear or branched hydrocarbon chain comprising from 1 to 22 carbon atoms;
- B is an optionally substituted nucleobase, selected from the group consisting of purine nucleobases, pyrimidine nucleobases, and non-natural monocyclic or bicyclic heterocyclic nucleobases wherein each cycle comprises from 4 to 7 atoms.

Preferably, the oligonucleotide sequence "Oligo-" is connected to the divalent linker moiety Y via a phosphate moiety -O-P(=O)(O⁻)-, at its 3' or 5' end, advantageously at its 5' end.

In a preferred embodiment according to the invention, the modified oligonucleotide is of the general formula (II'): 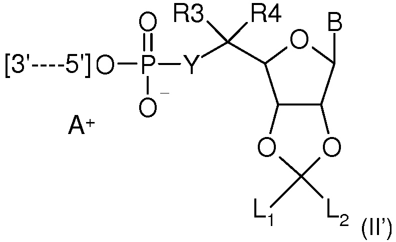 wherein:
- wherein Y, R₃, R₄, L₁, L₂ and B are as defined above in formula (II),
- [3'----5'] represents, along with the PO₃⁻ residue, an oligonucleotide as defined in the section *"Oligonucleotide"* herein above, and
- A⁺ represents a cation, preferably H⁺, Na⁺, K⁺ or NH₄⁺.

In the formulae (II) and (II'), the divalent linker moiety is preferably ether -O-.

In the formulae (II) and (II'), R₃ and R₄ are preferably hydrogen atoms.

In a preferred embodiment according to the invention, the modified oligonucleotide is of the formula (II"): 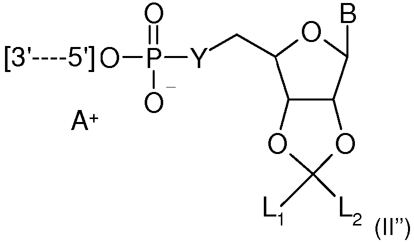 wherein Y, L₁, L₂ and B are as defined above in formula (II), A⁺ is a defined above in formula (II') and [3'--5'] represents, along with the PO₃⁻ residue, an oligonucleotide as defined in the section *"Oligonucleotide"* herein above.

In the formulae (II), (II') and (II"), L₁ and L₂ preferably represent a hydrocarbon chain, preferably a linear hydrocarbon chain, comprising from 6 to 22 carbon atoms, preferably from 8 to 18 carbon atoms, advantageously from 12 to 16 carbon atoms, more advantageously 15 carbon atoms.

In the formulae (II), (II') and (II"), B preferably represents a non substituted nucleobase selected from the group consisting of uracil, thymine, adenine, guanine, cytosine, 6-methoxypurine, 7-methylguanine, xanthine, 5,6-dihydrouracil, 5-methylcytosine, 5-hydroxymethylcytosine and hypoxanthine. Preferably, in the formulae (II), (II') and (II"), B represents a non substituted nucleobase selected from the group consisting of uracil, thymine, adenine, cytosine, 6-methoxypurine and hypoxanthine. More preferably, in the formulae (II), (II') and (II"), B represents uracil.

In a preferred embodiment according to the invention, the modified oligonucleotide is of the formula (II‴): 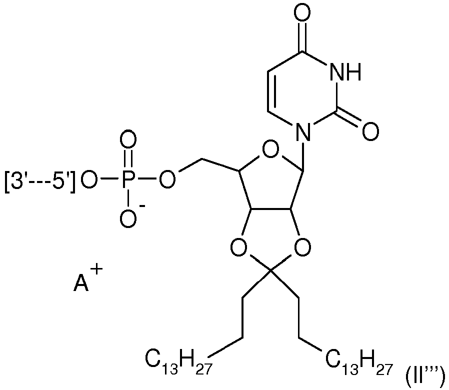 wherein A⁺ is as defined above in formula (II') and [3'--5'] represents, along with the PO₃⁻ residue, an oligonucleotide as defined in the section *"Oligonucleotide"* herein above.

### Tetramolecular parallel G-quadruplex

The present invention further concerns a tetramolecular parallel G-quadruplex comprising 4 identical modified oligonucleotides as defined above, wherein each of the 4 consecutive guanine residues included in the middle of the nucleic acid sequence of each oligonucleotide respectively form G-quartets with the corresponding guanine residues of the other 3 oligonucleotides, said G-quartets being stabilized by π-π staking and Hoogsteen hydrogen bonding.

By "tetramolecular parallel G-quadruplex" is meant herein a four-stranded nucleic acid structure, all four strands pointing in the same direction, comprising multiple stacked G-quartets, each of which consists of four guanine bases that associate in a cyclical manner through Hoogsteen hydrogen bonds and π-π staking and may be further stabilized though coordination to a cation in the center.

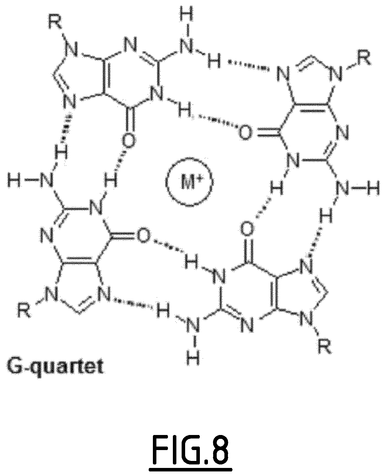

G-quartets coordinated to a cation are typically represented on **Figure 8****.**

In a preferred embodiment, the tetramolecular parallel G-quadruplex of the invention further comprises a cation, which preferably coordinates said G-quartets.

Said cation may be a monovalent or a divalent cation. Examples of suitable cations include K⁺ and Mg²⁺.

In a preferred embodiment, said cation is a divalent cation, more preferably a Mg²⁺ cation.

### Composition

The present invention also provides a composition comprising tetramolecular parallel G-quadruplexes as defined above, wherein the tetramolecular parallel G-quadruplexes self-assembled into micelles.

A micelle is an aggregate of surfactant molecules dispersed in a liquid colloid. A typical micelle in aqueous solution forms an aggregate with the hydrophilic "head" regions of the molecules in contact with surrounding aqueous solvent, sequestering the hydrophobic "tail" regions of the molecules in the micelle centre.

The tetramolecular parallel G-quadruplexes of the invention self-assemble into micelles having a core/shell structure, wherein the shell is hydrophilic and is formed of the oligonucleotide parts of the G-quadruplexes, and wherein the core is lipophilic and is formed of the lipid moiety of the modified oligonucleotides constituting the G-quadruplexes.

The compositions may comprise up to 50% by weight of modified oligonucleotides, preferably from 0.1% to 40%, in particular from 1% to 20%, and especially from 8% to 15% by weight of modified oligonucleotides.

According to a preferred embodiment of the composition of the invention, it further comprises a hydrophobic active principle hosted in said micelles.

Said micelles can be used for the loading of hydrophobic drugs. Loading of such micelles with hydrophobic drug can vary between 10 nM and 2 mM.

The hydrophobic active principle is preferably selected from the group consisting of Paclitaxel, Docetaxel, Vincristine, Vinorelbine, and Abraxane; Tamoxifen, Gonadotrophin-releasing hormone (GnRH) agonists and antagonists, androgen receptor (AR) antagonist, and estrogen receptor (ER) antagonists; Cyclophosphamide, Chlorambucil and Melphalan; Methotrexate, Cytarabine, Fludarabine, 6- Mercaptopurine and 5- Fluorouracil; Doxorubicin, Irinotecan, Platinum derivatives, Cisplatin, Carboplatin, Oxaliplatin; Bicalutamide, Anastrozole, Examestane and Letrozole; Imatinib (Gleevec), Gefitinib and Erlotinib; Rituximab, Trastuzumab (Herceptin) and Gemtuzumab ozogamicin; Interferon-alpha; Tretinoin and Arsenic trioxide; Bevicizumab, Serafinib and Sunitinib.

As shown in the example, the stability of the G4 and/or the micelles according to the invention can be modulated playing around 1) the nature of the lipid moiety, 2) the nature of the salts present in solution (with an emphasis on Mg²⁺) and 3) the presence of a G4-prone segment within the oligonucleotide sequence.

### Use as vehicle

The present invention also concerns the use of a composition as defined in the section *"Composition"* hereabove as a vehicle.

As used herein, the term "vehicle" refers to a carrier of a medicinally and/or pharmaceutically active substance.

Preferably, the composition as defined in the section *"Composition"* hereabove is used as a carrier of sparingly hydrosoluble active principle.

Such a vehicle is notably useful for the administration of such active substance by way of intravenous, intraperitoneal, subcutaneous or oral routes, or direct hemoral injection.

The active principle is preferably selected from the group consisting of Paclitaxel, Docetaxel, Vincristine, Vinorelbine, and Abraxane; Tamoxifen, Gonadotrophin-releasing hormone (GnRH) agonists and antagonists, androgen receptor (AR) antagonist, and estrogen receptor (ER) antagonists; Cyclophosphamide, Chlorambucil and Melphalan; Methotrexate, Cytarabine, Fludarabine, 6- Mercaptopurine and 5- Fluorouracil; Doxorubicin, Irinotecan, Platinum derivatives, Cisplatin, Carboplatin, Oxaliplatin; Bicalutamide, Anastrozole, Examestane and Letrozole; Imatinib (Gleevec), Gefitinib and Erlotinib; Rituximab, Trastuzumab (Herceptin) and Gemtuzumab ozogamicin; Interferon-alpha; Tretinoin and Arsenic trioxide; Bevicizumab, Serafinib and Sunitinib.

### Medical indications

The present invention also concerns the composition as defined in the section *"Composition"* hereinabove for use as a medicament.

The invention also concerns the use of the composition as defined in the section *"Composition"* hereinabove for the manufacture of a medicament.

The present invention further concerns a method for treating a subject in need thereof, comprising administering to a subject in need thereof a therapeutically effective amount of a composition as defined in the section *"Composition"* hereinabove.

The composition is administered in an "effective amount", *i.e.* in an amount sufficient to treat the subject in need thereof. It will be appreciated that this amount will vary both with the effectiveness of the composition, in particular of the active principle hosted in the micelles of the composition, or other therapeutic agent employed, and with the nature of any carrier used. The determination of appropriate amounts for any given composition is within the skill in the art, through standard series of tests designed to assess appropriate therapeutic levels.

In the frame of the present invention, the individual preferably is a human individual. However, the veterinary use of the composition according to the present invention is also envisioned. The individual may thus also correspond to a non-human individual, preferably a non-human mammal.

The term "treating" is meant a therapeutic method, *i.e.* a method aiming at curing, improving a disease and/or extending the lifespan of an individual suffering from a disease.

### Use in nanotechnology

The present invention further concerns the use of a tetramolecular parallel G-quadruplex as defined above in the manufacture of nanotechnology devices.

In particular the tetramolecular parallel G-quadruplex as defined above may be used for the manufacture of nanostructures, of biosensors or nanomachines, of gels or films, and/or of organic semiconductors. For example, the tetramolecular parallele G-quadruplex as defined above may be used for the manufacture of nanowires, ion channels, stationary phases in chromatography or electrophoresis, gels, films and/or organic semi-conductors.

The present invention also concerns a method of manufacture of nanotechnology devices, such as nanostructures, biosensors or nanomachines, gels or films, and/or organic semiconductors, in particular nanowires, ion channels, stationary phases in chromatography or electrophoresis, gels, films and/or organic semi-conductors, said method comprising the use of a tetramolecular parallel G-quadruplex as defined above.

The tetramolecular parallel G-quadruplexes as defined above may also be used as artificial implants.

Accordingly, the present invention further concerns the use of a tetramolecular parallel G-quadruplex as defined above in the manufacture of artificial implants.

Another object of the invention relates to the tetramolecular parallel G-quadruplex as defined above for use as artificial implant.

The present invention will be further described by way of the examples below and the drawings in annex.

### Brief description of the sequences

SEQ ID NO: 1 shows the sequence of a modified oligonucleotide according to the invention.

SEQ ID NO: 2 shows the sequence of the "scramble" oligonucleotide used in the example.

### Brief description of the figures

**Figure 1****:** Native PAGE of the different (L)ONs synthesized in the example in the presence of 1X salts (except lane 3). The absence of band for k-LON^{G4} in lane 7 results from the formation of stable micellar aggregates (the other micellar aggregates do not survive the PAGE conditions). 1X salts = 50 mM Nacl, 5mM KCI, 5mM MgCl₂.
**Figure 2****:** CD/NMR signatures of 1-LON^{G4}, 1-LON^{SC}, ON^{G4} and ON^{SC} of the example (Conditions: [LON] = 5 µM, diluted from original 30 µM solutions, 1X salts, phosphate buffer pH 6.9, 20 mM).
**Figure 3****:** Gel retardation of 1-LON^{G4}. Lane 1: no added salt; Lane 2: 1X; Lane 3: "LiCI": 55mM LiCl, 5mM MgCl₂; Lane 4: 4X; Lane 5: LiCI 4X; Lane 6: 4X; Lane 7: KCI 0.3M; Lane 8: KCI 1.2M; Lane 8: AcONH₄ 0.05M; Lane 9: AcONH₄ 0.2M; Lane 10: control intramolecular 77-mer G4.
**Figure 4****:** Native agarose gel of the different (L)ONs studied in the example (see Figure 1 for salt conditions).
**Figure 5****:** DLS/TEM analysis of 1-LON^{G4} of the example.
**Figure 6****:** Effect of temperature cycles form 1-LON^{G4}.
**Figure 7****:** Conceptual scheme showing the lipid-driven assembly of large tetramolecular parallel G-quadruplexes.
**Figure 8****:** Chemical scheme of a G-quartet.

### Example

This example shows that the lipid modification of a G4 prone oligonucleotide sequence with lipids drastically increases the probability of forming tetramolecular parallel G-quadruplexes over other unspecific oligomers.

### Materials and methods

### 1. Automated DNA synthesis and purification of LONs

LONs were synthesized using the phosphoramidite methodology on an automated Expedite 8909 DNA synthesizer at the µmol scale on 1000 Å primer support (loading: 30-100 µmol/g, Link technologies, Synbase Control Pore Glass). Prior to use, the phosphoramidites **1** of formula (III) 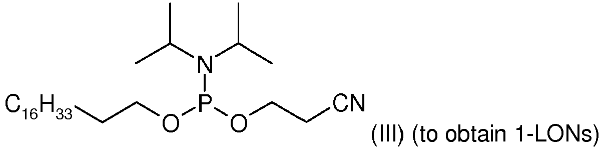 and **2** of formula (IV) 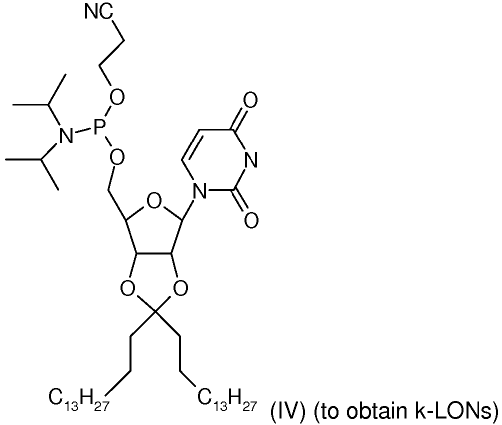 were dried over P₂O₅ overnight and then dissolved in dry CH₂Cl₂/CH₃CN 1/1 to a 0.1 M concentration (lipidic phosphoramidites did not dissolve in pure acetonitrile). N-benzylthiotetrazole was used for activation of the phosphoramidite prior to coupling. The phosphoramidites **1** and **2** were manually coupled last on the solid support by passing (via syringes) the activator and the phosphoramidite solution (0.25 mL) back and forth several times for 7 min. Deblocking and detachment from the solid support was achieved using 1 mL of a saturated aqueous NH₄OH solution for 12 h at 55°C. The supernatant was collected and the CPG beads were washed 3 times with 0.25 mL of EtOH/CH₃CN/H₂O 3/1/1 (vol). The solutions were pooled and evaporated (speed vac). The crude LONs were dissolved in 0.3 mL of water and purified (except the k-LONs) on an analytical C4-reverse phase HPLC using buffer A (0.1 M triethylammonium acetate, pH 6.5) with 20 % of buffer B (0.1 M triethylammonium acetate, pH 7.0, 80 % acetonitrile) over 2 min, followed by buffer B over 50 min (flow rate: 2 ml/min). The product oligoamphiphiles eluted after ca. min. Product containing fractions were pooled and evaporated to dryness and dissolved in autoclaved milliQ water. The (L)ONs were then dialyzed against a 10 mM LiCI solution (not to favor G4 formation) followed by water. Yields of recovery were acceptable following this protocol (15-30% yield after purification of the crude material). Yields of final LON^{G4} (ε=182800) and LON^{SC} (ε=184600).

k-LONs were purified by preparative PAGE using conventional protocols with 20*20*0.2 cm 20% polyacrylamide gels at a limiting power = 15W. Importantly, the inventors found that the quantity of k-LON^{G4} that could be loaded on the gel did not exceed ca. 100 nmoles of crude material in ca. 300µL of loading sample. Higher amounts of crude LON led to substantial trailing of the LON band probably because of the formation of the micelles in that case even in the presence of heat + 7M urea in the running buffer. Using smaller quantities, the k-LON^{G4} band is well defined (UV-shadow) and cut out directly with a clean scalpel. The gel slab was chopped into fine particles to elute the LON. The inventors have been unsuccessful at eluting k-LON^{G4} from the gel using different eluting buffers with additional heating (up to 90°C) and/or sonication and/or freeze and thaw protocols. Small quantities of k-LON^{G4} were systematically obtained for each of these tests. The inventors therefore developed an original electroelution protocol for the purification of these LONs. In short, the electrical wire that was plugged in the negative pole of the generator was manually modified by wrapping a platinum wire around the naked copper wire. The latter was immersed in the eluting buffer contained in a plastic pipette that was chopped off at both edges to 1) facilitate pouring of the eluting buffer on top and 2) increase the cross section at the bottom to minimize resistance to the current flow. The bottom of the syringe was blocked by polymerizing 0.5 mL of a 8% polyacrylamide solution (the bottom of the pipette being temporarily blocked by wrapping a parafilm foil around). After polymerization, the gel was prerun to remove any unpolymerized materials prior to loading the crushed acrylamide gel slab containing the LON (the TBE eluting buffer from the pipette can be withdrawn beforehand for practical reasons). A dialysis tubing (with a cutoff of 2 kDa) was adapted and wrapped with a parafilm foil around the bottom of the pipette to recover the sample after electroelution. This allowed the dialysis tubing to be immersed in a large quantity of eluting buffer at the bottom in the electrophoresis tank. The elution was carried out with an electrical power of 7-10 W to allow enough heat dissipation in the gel to favor denaturation of the LONs. The LONs were finally dialyzed in a similar manner as described above for the other (L)ONs.

### 2. Mass spectra measurements

Mass spectra were recorded on a MALDI-Tof-ToF mass spectrometer (Ultraflex, Bruker Daltonics, Bremen, Germany). Best results were obtained in the linear mode with positive-ion detection. Mass spectra were acquired with an ion source voltage 1 of 25kV, an ion source voltage 2 of 23.5kV, a lens voltage of 6kV, by accumulating the ion signals from 1000 laser shots at constant laser fluence with a 100Hz laser. External mass calibration was achieved using a mixture of oligonucleotides dT₁₂-dT₁₈ (Sigma). 1:1 mixture of samples of LONs (~20-50µM) and matrix was spotted on a MALDI target and air-dried before analysis. The performance of the following matrices was evaluated: 2,5-dihydroxybenzoic acid (DHB), 2,4,6-trihydroxy-acetophenone (THAP), 3-hydroxypicolinic acid (3-HPA) and 2,6-dihydroxyacetophenone (DHA). THAP at a concentration of 20 mg/mL in a 4:1 mixture of ethanol and 100mM aq ammonium citrate was shown to yield optimal mass spectral results.

MALDI-TOF mass analyses:
1-LON^{G4}: [M-H]- calculated MW: Da, found: Da (0.0% error);
1-LON^{SC}: [M-H]- calculated MW: Da, found: Da (0.0% error).
k-LON^{G4}: [M-H]- calculated MW: Da, found: Da (0.0% error);
k-LON^{SC}: [M-H]- calculated MW: Da, found: Da (0.0% error).
ON^{SC}: [M-H]- calculated MW: 5920.9 Da, found: 5924.29 Da (0.056% error).
ON^{G4}: [M-H]- calculated MW: 5920.9 Da, found: 5924.22 Da (0.057% error).

### 3. PAGE / agarose electrophoresis

Electrophoresis experiments were performed according to standard procedures with 1% agarose gels. PAGE were carried out with 17% polyacrylamide gels and run with a 100V limiting tension for native PAGE experiments.

### 4. Dynamic light scattering (DLS)

Particle size was determined using a Zetasizer 3000 HAS MALVERN. Experiments were realized with samples containing different concentration of LONs dissolved in deionized water or phosphate buffer. Measurements were performed at 25°C.

### 5. Taylor Dispersion Analysis (TDA, discosizing)

TDA analyses were recorded on a Viscosizer TD (Malvern Instruments Ltd., Malvern, UK equipped with a 254 nm UV filter close to the λₘₐₓ of oligonucleotides. Prior to analysis, the non-coated capillary has been prepared by injecting 1M NaOH during 30 min at 3000 mbar followed by 10 min rinse with water at 3000 mbar. The cellulose coated capillary has been prepared by injecting water during 30 min at 3000 mbar.

Internal Material (non-coated): fused silica, internal diameter: 75 µm, outer diameter: 360 µm, L1: 45 cm, L2: 85cm, Total Length: 130 cm.

The samples were injected (pressure: 50 mbar), analyzed at 25°C (Mobilization pressure: 140 mbar). Taylorgramms were recorded and analyzed with the viscosizer TD software 2.01 with a one component fit. Washing: 1 min of water between each sample, pressure: 3000 mbar.

The coated cellulose capillary was in general necessary for the analysis of LONs with the noticeable exception of k-LON^{G4}. Unless the LON forms stable micellar assemblies as in the case of k-LON^{G4}, unspecific adsorption was observed onto the uncoated capillary with the other LONs as evidenced by a trailing in the absorption curve of the chromatogram.

### 6. TEM

TEM analyses were performed at the Bordeaux Imaging Center (BIC) of the University of Bordeaux using an Hitachi H7650 at a voltage of 80 kV. For sample preparation a drop of 100 nM solution of 1-LON^{G4}, 2X selex salts was placed on a carbon film 200 Mesh copper grid and left to dry for 10 min. A drop of 1% uranyl acetate solution as a negative stain for 1 min was then added to the copper grid and left to dry.

### Results

The oligonucleotide sequence used by the inventors was chosen to embark a G-tract of 4 consecutive guanines in the middle of a 19-mer DNA sequence (LON^{G4}).

The DNA molecule was modified at the 5'-end with different lipid phosphoramidites **(Table** 1).

**Table 1: Phosphoramidites used in the example**

| **Name** | **Lipid moiety** | **Lipidic phosphoramidite used** |
|---|---|---|
| **ON** | None (5'-OH) | None |
| **1-LON** | *n*-C₁₈H₃₇ | 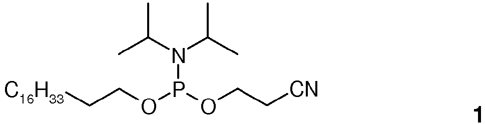 |
| **k-LON** | Ketal bis-C₁₅ | 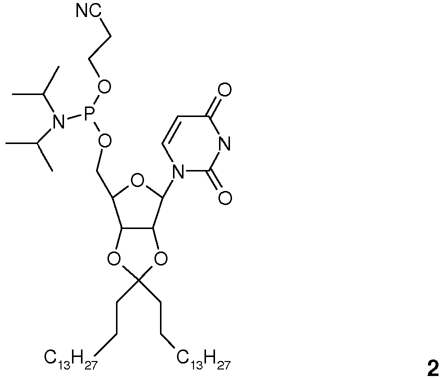 |

The phosphoramidites 1 and 2 were synthesized according to literature procedures and then coupled to the 5'-end of the DNA using an automated solid phase DNA synthesizer. The randomized LON sequences (scramble - LON^{SC}) were also synthesized as controls wherein the guanines were evenly distributed among the whole oligonucleotide sequence to minimize the chance of forming undesired foldings.

In summary, the following optionally lipid-modified oligonucleotides were synthesized:
**ON^{G4} :** 5'-TTA GTT GGG GTT CAG TTG G-3' (SEQ ID NO: 1)
**ON^{SC} :** 5'-TGT AGT AGG TTG TGT CTG G-3' (SEQ ID NO: 2)
**1-LON^{G4}** : *n*-C₁₈H₃₇-5'-TTA GTT GGG GTT CAG TTG G-3' (SEQ ID NO: 1)
**1-LON^{SC}** : *n*-C₁₈H₃₇-5'-TGT AGT AGG TTG TGT CTG G-3' (SEQ ID NO: 2)
**k-LON^{G4}** : ketal-5'-TTA GTT GGG GTT CAG TTG G-3' (SEQ ID NO: 1)
**k-LON^{SC}** : ketal-5'-TGT AGT AGG TTG TGT CTG G-3' (SEQ ID NO: 2)

The purification of these LONs offered a first insight at their self-assembling properties. When the lipid is attached at the 5'-end of the oligonucleotide, the capping step during the ON synthesis precludes abortive sequences from reacting with the lipid phosphoramidite. Consequently, the purification of these LONs is theoretically straightforward as only the desired full length ON is coupled to the lipid and somewhat interacts with the reverse stationary phase. While the control k-LON^{SC} was readily purified by RP (C₄)-HPLC, the majority of the k-LON^{G4} eluted in the dead volume after the first injection of the crude mixture. Surprisingly, more k-LON^{G4} was eluted in the second HPLC run when water alone was injected. The di-alkylated LONs likely form stable aggregates whose lipid segments are buried inside the aggregate preventing them from interacting with the stationary phase (vide infra). An original polyacrylamide gel electrophoresis (PAGE) protocol for the purification of k-LON^{G4} was therefore developed.

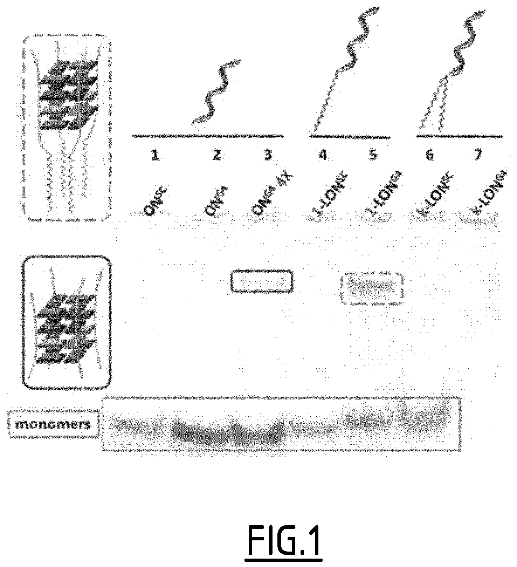

The nature of the supramolecular assemblies formed from these different LONs was investigated by non-denaturing PAGE. Interestingly, both the sequence (G4 or scramble) and the nature of the lipid were shown to impact the self-assemblies formed from these LONs **(****Figure 1****).**

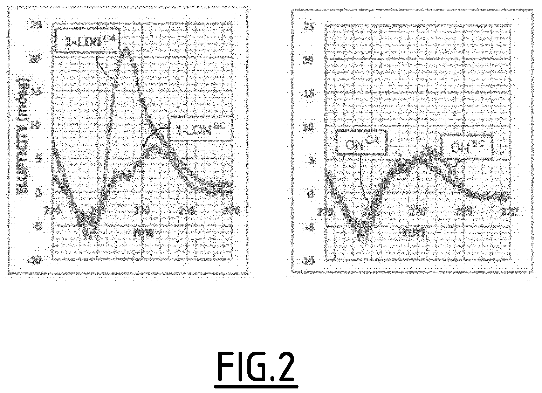

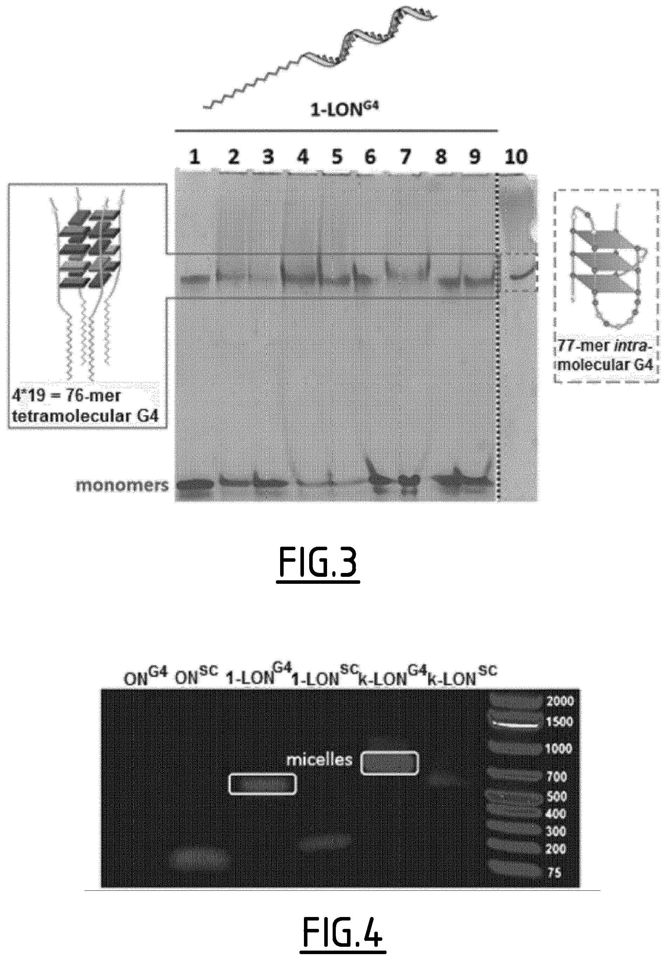

As expected, no clear foldings were observed for the different scramble LONs (lanes 1, 4 and 6). Surprisingly, no G4 structures were visible as well with the unmodified ON^{G4} (lane 2), probably because of the length of the oligonucleotide that precludes the correct folding of the G-quadruplex. In contrast, the presence of the octadecyl chain in 1-LON^{G4} resulted in the appearance of a retarded band (lane 5) corresponding to a tetramolecular parallel G-quadruplex as judged by its CD/NMR signature and its retardation in the gel **(****Figures 2** **and** **3****).** A similarly retarded faint band was visible with the unmodified ON^{G4} only when the salt concentration was increased (lane 3 of **Figure 1****).** Of note, the inventors clearly ruled out the possibility of a kinetic control over the equilibrium of formation of the tpG4. The ratio between the monomer and the tpG4 for both 1-LON^{G4} and ON^{G4} were clearly time independent (up to 4 weeks) as judged by PAGE and CD.

While the proportion of 1-LON^{G4} molecules that form tpG4 fold quickly (t < 5 mins for CD experiments), the other 1-LON^{G4} molecules are trapped in undesired foldings (vide infra). Again, the k-LON^{G4} LON modified with a double chain lipid exhibited a peculiar behavior. The micellar aggregates that are formed from this LON are stable and large enough to survive the electrophoresis conditions and to prevent migration within the reticulated acrylamide polymer (lane 7 of **Figure 1****).** This result clearly illustrated the importance the oligonucleotide sequence has on the self-assembling process as the control k-LON^{SC} migrated normally in the gel (lane 6 of **Figure 1****).**

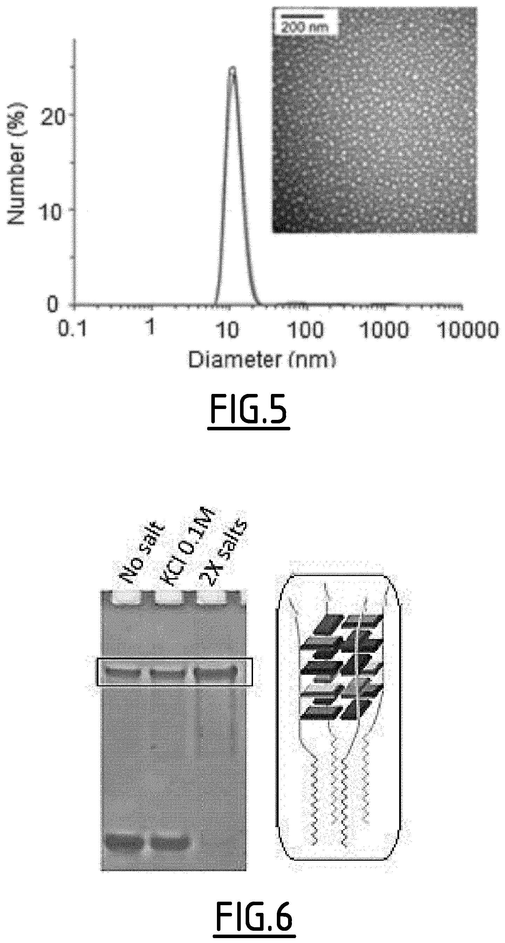

Given the relatively small size and volume of the lipid segment of the LONs compared to the large DNA polar heads, the LONs used for these investigations were expected to form micellar aggregates. Indeed, dynamic light scattering (DLS) and Taylor Dispersion Analysis (TDA) experiments confirmed these expectations. These results suggest that the G4-prone forming sequence of k-LON^{G4} greatly stabilizes the micelles. The kinetic stability of k-LON^{G4} micelles over those formed from k-LON^{SC} was further evidenced by the partitioning and the lack thereof of k-LON^{SC} and k-LON^{G4} monomers respectively into micelles of the neutral triton detergent as well as the absence of any micelle disassembly in the presence of acetone. On the other hand, the presence of the G4-prone forming sequence in the LON sequence did not necessarily translate into stable micellar aggregates as evidenced by the migration observed with 1-LON^{G4} **(****Figure 1****,** lane 5). The reticulated sieving acrylamide matrix is prone to disassemble loose micellar aggregates especially in the presence of an efficient divalent cation scavenger like EDTA present in the PAGE experiment. Micelles of 1-LON^{G4} were nevertheless present in solution as evidenced by agarose gel **(****Figure 4****,** lane 3), DLS and TEM **(****Figure 5****).** Interestingly, no micellar aggregates were observed with the control 1-LON^{SC} **(****Figure 4****,** lane 4). Consequently, tpG4-mediated micelle stabilization was still at play with 1-LON^{G4}. The tpG4 monomer of 1-LON^{G4} partitioned with the ones in the micelle in that case. Agarose gel results also confirmed the weak tendency of unmodified ON^{G4} to form G4 structures, only a faint retarded band being visible in the gel **(****Figure 4****,** first lane). Interestingly, k-LON^{G4} exhibited well-defined micellar bands compared to their scramble analogs **(****Figure 1****,** lanes 5 and 6 respectively). No voluminous aggregates being detectable by DLS and Taylor dispersion analyses, the prominent trailing shoulders observed with k-LON^{SC} more likely resulted from unspecific adsorptive interactions between the highly concentrated free scramble DNA at the micelle surface and the agarose gel matrix during electrophoresis. These unspecific interactions have been shown to increase with DNA sizes and decrease with increasing salt concentrations, in line with the present observations. Conversely, favorable intra-micellar G4 formation in k-LON^{G4} micelles may prevent unspecific adsorption of the LON micelles with the agarose matrix.

Furthermore, the inventors observed a noticeable retardation with k-LON^{G4} compared to k-LON^{SC}. The G4-prone sequences may indeed be poised to adopt a brush-like regime at the surface of the micelle upon formation of the extended and rigid intra-micellar and parallel G-quadruplexes with a concomitant increase in micellar size and/or change in morphology, or aggregation number compared to the looser scramble sequences. DLS and Taylor Dispersion Analysis (TDA) of these samples fully supported native PAGE and agarose observations. For instance, micelles of 1-LON^{SC} were only visible by DLS at concentration above 50 µM compared to the 20 µM used in the PAGE experiments and the size of k-LON^{G4} micelles was superior to k-LON^{SC}. It is worth mentioning that even though no discrete parallel G-quadruplexes were observed with k-LON^{G4}, quadruplex formation at the surface of the micelle still occurred as judged by their CD signature and the K⁺-dependence observed in agarose gel.

All tetramolecular G-quadruplexes reported in the literature so far were parallel and were formed from short oligonucleotide sequences, most probably because of polymorphism issues. Given their molecularity of four, their kinetics of formation are extremely slow at micromolar concentrations. Yet, once formed, these aggregates are very stable: no melting is observed at T > 95°C provided a minimum amount of K⁺ is present in solution. To the inventor's knowledge, the tpG4 from 1-LON^{G4} is the first tpG4 of moderate size reported. Besides, the inventors found that this G4 melted at a reasonable temperature (Tm = 79°C). The decrease in the melting temperature of 1-LON^{G4} tpG4s compared to shorter ones may result from the long flanking probably disordered DNA sequences. The thermal reversibility of tpG4 formation constitutes a clear advantage for the design of switchable nano-devices for biotechnological applications.

Although, a single lipid chain was effective at promoting tpG4 formation from 1-LON^{G4}, monomers were still apparently visible **(****Figure 1****,** lane 5). Given the high thermal stability of 1-LON^{G4} tpG4 and the absence of change in the ratio 1-LON^{G4} monomer / tpG4 with time (see above), no equilibrium exists between these 2 entities. In fact, the inventors found that the alleged "monomers" of 1-LON^{G4} were not free to equilibrate in solution with the tpG4. Given its high thermal stability, the 1-LON^{G4} tpG4 corresponds to an energetic minimum in the different energetic pathways that lead to other undesired foldings in solution. This was first checked by running a bi-dimensional native PAGE with 1-LON^{G4}: the tpG4 band showed no trace of equilibrium in the second dimension (no salt). Instead, the remaining "monomers" of 1-LON^{G4} in solution were kinetically trapped in less stable, undesired foldings and/or aggregates. This hypothesis was confirmed by heating the solution above the melting temperature of the undesired foldings but below the Tm of the tpG4. The freed monomers were again available to form more tpG4s upon cooling. Several temperature cycles were required to enrich the solution in the desired tpG4 **(****Figure 6****).**

Very importantly, while temperature cycles applied to 1-LON^{G4} in the absence of salt or in the presence of KCI had no or little influence on the formation of the tpG4 **(****Figure 6****,** 2 first lanes), Mg²⁺ was found to be very important for the correct folding of 1-LON^{G4} into tpG4 **(****Figure 6****,** last lane). This result came as a real surprise as K⁺ is the cation of choice for the stabilization of G-quadruplexes. Instead, the inventors found that Mg²⁺ remarkably stabilized all the LON micellar assemblies irrespective of their oligonucleotide sequence (G4 or scramble). For instance, Mg²⁺ was necessary to observe 1-LON^{G4} micelle in agarose gel **(****Figure 4****)** and no more migration of the k-LON^{SC} was observed in PAGE with increased amounts of Mg²⁺ as a result of the higher stability of the micelles, just as what was observed in the absence of salts with k-LON^{G4} **(****Figure 1****,** lane 7). These explanation of the inventors for this impressive magnesium effect is that only the desired tpG4 is capable of forming stable micellar assemblies that are further stabilized by Mg²⁺ bridging between quadruplexes. Following the Le Chatelier's principle, the tpG4s are withdrawn from the equilibrium to form the stable micelle and provided the unspecific other aggregates are heated above their melting temperature, more tpG4s are formed at each heating and cooling cycle.

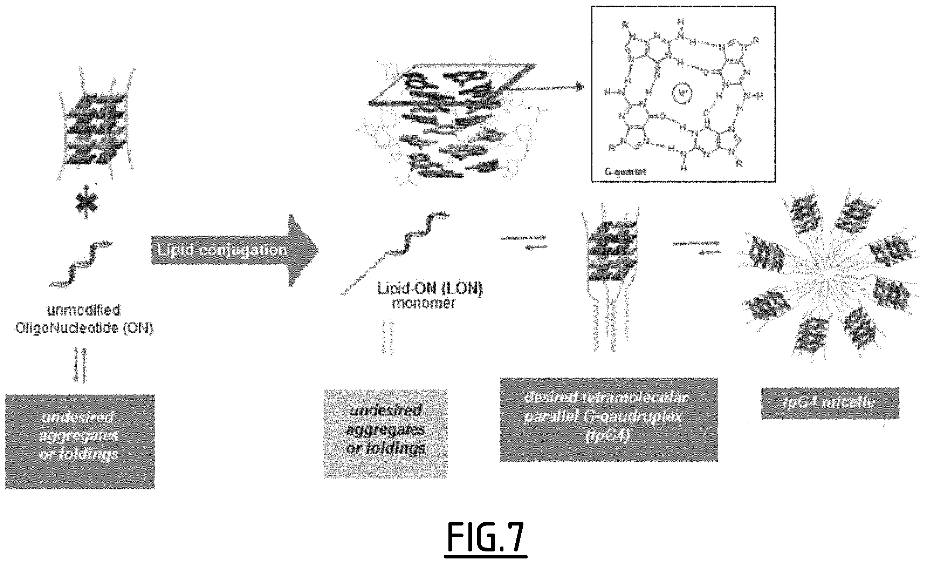

In conclusion the inventors have shown that the modification of a G4-prone oligonucleotide sequence with lipids drastically increases the probability of forming tetramolecular parallel G-quadruplexes over other undesired foldings or oligomers. These results indicate that lipids constitute key elements in the supramolecular organization of G4-prone LONs. They first serve as a guide for the correct folding of the LON into the desired G4 over other undesired aggregates. The G4 segment in return (together with Mg²⁺) are crucial for the stabilization of the micellar systems that eventually lead to the enrichment in tpG4 provided heating and cooling cycles are applied to the solution. This lipid-driven assembly of tpG4 is schemed on **Figure 7****.** The stability of the micellar aggregates can be modulated playing around 1) the lipid, 2) the nature of the salts present in solution (with an emphasis on Mg²⁺) and 3) the presence of a G4-prone segment within the oligonucleotide sequence of the LON. These data highlight the potential of LON for the construction of parallel G-quadruplexes of unprecedented length. This provides a means to design and construct promising potentially switchable supramolecular architectures for nanotechnology and nucleic acid-based therapeutics.

## Claims

1. An oligonucleotide modified by substitution at the 5' or the 3' end by a lipid moiety,, of the general formula (II) 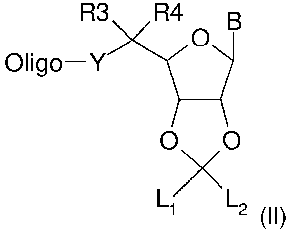 wherein:
• Oligo represents a nucleic acid sequence of at least 10 nucleotides, said nucleic acid sequence including a series of at least 4 consecutive guanine residues located in the middle of said sequence, i.e. wherein the series of guanine residues is not located at the 5' end or at the 3' end of the nucleic acid sequence, and is separated from each end of the nucleic acid sequence by a number of nucleotides corresponding to at least 25% of the total number of nucleotides of the nucleic acid sequence , the number of nucleotides separating the series of guanine from the 5' end and the 3' end of the nucleic acid sequence being identical or different, wherein said nucleic acid sequence may be oriented 3'-5' or 5'-3' and/or may comprise modified nucleotides;
• Y represents a divalent linker moiety selected from ether -O-, thio - S-, amino -NH-, and methylene -CH₂-;
• R₃ and R₄ may be identical or different and represent:
∘ a hydrogen atom,
∘ a halogen atom,
∘ a hydroxyl group, or
∘ an alkyl group comprising from 1 to 12 carbon atoms;
• L₁ and L₂ may be identical or different and represent a saturated or unsaturated, linear or branched hydrocarbon chain comprising from 1 to 22 carbon atoms;
• B is an optionally substituted nucleobase, selected from the group consisting of purine nucleobases, pyrimidine nucleobases, and non-natural monocyclic or bicyclic heterocyclic nucleobases wherein each cycle comprises from 4 to 7 atoms.

2. The modified oligonucleotide according to claim 1, wherein said oligonucleotide consists of a DNA sequence of 19 nucleotides.

3. The modified oligonucleotide according to any one of claims 1 to 2, wherein said oligonucleotide consists of the sequence 5'-TTAGTTGGGGTTCAGTTGG-3' (SEQ ID NO: 1).

4. A tetramolecular parallel G-quadruplex comprising 4 identical modified oligonucleotides as defined in any one of claims 1 to 3, wherein each of the 4 consecutive guanine residues included in the middle of the nucleic acid sequence of each oligonucleotide respectively forms G-quartets with the corresponding guanine residues of the other 3 oligonucleotides, said G-quartets being stabilized by π-π stacking and Hoogsteen hydrogen bonding.

5. The tetramolecular parallel G-quadruplex according to claim 4, wherein said G-quadruplex further comprises a divalent cation which coordinates said G-quartets.

6. The tetramolecular parallel G-quadruplex according to claim 5, wherein said divalent cation is a Mg²⁺ cation.

7. A composition comprising tetramolecular parallel G-quadruplexes according to any one of claims 4 to 6, wherein the tetramolecular parallel G-quadruplexes self-assembled into micelles.

8. The composition according to claim 7 further comprising a hydrophobic active principle hosted in said micelles.

9. Use of the composition according to claim 7 or 8 as a vehicle.

10. Composition according to claim 7 or 8 for use as a medicament.

11. Use of a tetramolecular parallel G-quadruplex according to any one of claims 4 to 6 in the manufacture of nanotechnology devices.

12. Tetramolecular parallel G-quadruplex according to any one of claims 4 to 6 for use as artificial implant.

## Patentansprüche

1. Oligonukleotid, modifiziert durch Substitution an dem 5'- oder 3'-Ende durch eine Lipidgruppierung, der allgemeinen Formel (II) 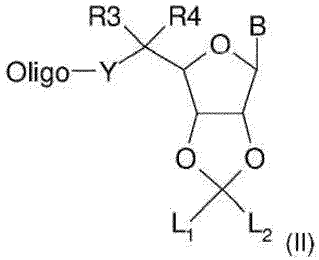 wobei:
• Oligo eine Nukleinsäuresequenz von mindestens 10 Nukleotiden darstellt, wobei die Nukleinsäuresequenz eine Reihe von mindestens 4 aufeinanderfolgenden Guaninresten beinhaltet, die sich in der Mitte der Sequenz befinden, d. h. wobei sich die Reihe von Guaninresten nicht an dem 5'-Ende oder an dem 3'-Ende der Nukleinsäuresequenz befindet und von jedem Ende der Nukleinsäuresequenz durch eine Anzahl von Nukleotiden getrennt ist, die mindestens 25 % der Gesamtanzahl von Nukleotiden der Nukleinsäuresequenz entspricht, wobei die Anzahl von Nukleotiden, die die Guaninreihe von dem 5'-Ende und dem 3'-Ende der Nukleinsäuresequenz trennen, gleich oder verschieden ist, wobei die Nukleinsäuresequenz 3'-5' oder 5'-3' ausgerichtet sein kann und/oder modifizierte Nukleotide umfassen kann;
• Y eine zweiwertige Linkereinheit darstellt, ausgewählt aus Ether -O-, Thio - S-, Amino -NH- und Methylen -CH₂-;
• R₃ und R₄ gleich oder verschieden sein können und Folgendes darstellen:
∘ ein Wasserstoffatom,
∘ ein Halogenatom,
∘ eine Hydroxylgruppe, oder
∘ eine Alkylgruppe, umfassend 1 bis 12 Kohlenstoffatome;
• L₁ und L₂ gleich oder verschieden sein können und eine gesättigte oder ungesättigte, lineare oder verzweigte Kohlenwasserstoffkette darstellen, umfassend 1 bis 22 Kohlenstoffatome;
• Beine optional substituierte Nukleobase ist, ausgewählt aus der Gruppe, bestehend aus Purin-Nukleobasen, Pyrimidin-Nukleobasen und nicht natürlichen monocyclischen oder bicyclischen heterocyclischen Nukleobasen, wobei jeder Zyklus 4 bis 7 Atome umfasst.

2. Modifiziertes Oligonukleotid nach Anspruch 1, wobei das Oligonukleotid aus einer DNA-Sequenz von 19 Nukleotiden besteht.

3. Modifiziertes Oligonukleotid nach einem der Ansprüche 1 bis 2, wobei das Oligonukleotid aus der Sequenz 5'-TTAGTTGGGGTTCAGTTGG-3' (SEQ ID NO: 1) besteht.

4. Tetramolekularer paralleler G-Quadruplex, umfassend 4 identische modifizierte Oligonukleotide wie definiert in einem der Ansprüche 1 bis 3, wobei jeder der 4 aufeinanderfolgenden Guaninreste, die in der Mitte der Nukleinsäuresequenz von jedem Oligonukleotid beinhaltet sind, jeweils G-Quartette mit den entsprechenden Guaninresten der anderen 3 Oligonukleotide bildet, wobei die G-Quartette durch -π-π-Aufschichten und Hoogsteen-Wasserstoffbindungen stabilisiert sind.

5. Tetramolekularer paralleler G-Quadruplex nach Anspruch 4, wobei der G-Quadruplex ferner ein zweiwertiges Kation umfasst, das die G-Quartette koordiniert.

6. Tetramolekularer paralleler G-Quadruplex nach Anspruch 5, wobei das zweiwertige Kation ein Mg²⁺-Kation ist.

7. Zusammensetzung, umfassend tetramolekulare parallele G-Quadruplexe nach einem der Ansprüche 4 bis 6, wobei die tetramolekularen parallelen G-Quadruplexe sich selbst zu Mizellen organisieren.

8. Zusammensetzung nach Anspruch 7, ferner umfassend einen hydrophoben Wirkstoff, der in den Mizellen gehostet ist.

9. Verwendung der Zusammensetzung nach Anspruch 7 oder 8 als Vehikel.

10. Zusammensetzung nach Anspruch 7 oder 8 zur Verwendung als Medikament.

11. Verwendung eines tetramolekularen parallelen G-Quadruplexes nach einem der Ansprüche 4 bis 6 bei der Herstellung von Nanotechnologie-Vorrichtungen.

12. Tetramolekularer paralleler G-Quadruplex nach einem der Ansprüche 4 bis 6 zur Verwendung als künstliches Implantat.

## Revendications

1. Oligonucléotide modifié par substitution au niveau de l'extrémité 5' ou 3' par un fragment lipidique, de formule générale (II) 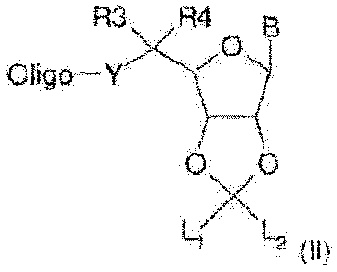 dans lequel :
• Oligo représente une séquence d'acide nucléique d'au moins 10 nucléotides, ladite séquence d'acide nucléique comprenant une série d'au moins 4 résidus de guanine consécutifs situés au milieu de ladite séquence, c-à-d. dans lequel la série de résidus de guanine n'est pas située au niveau de l'extrémité 5' ou au niveau de l'extrémité 3' de la séquence d'acide nucléique, et est séparée de chaque extrémité de la séquence d'acide nucléique par un nombre de nucléotides correspondant à au moins 25 % du nombre total de nucléotides de la séquence d'acide nucléique, le nombre de nucléotides séparant la série de guanine de l'extrémité 5' et de l'extrémité 3' de la séquence d'acide nucléique étant identique ou différent,
dans lequel ladite séquence d'acide nucléique peut être orientée 3'-5' ou 5'-3' et/ou peut comprendre des nucléotides modifiés ;
• Y représente un fragment de liaison divalent choisi parmi un éther -O-, un thio - S-, un amino -NH- et un méthylène -CH₂- ;
• R₃ et R₄ peuvent être identiques ou différents et représentent :
∘ un atome d'hydrogène,
∘ un atome d'halogène,
∘ un groupe hydroxyle, ou
∘ un groupe alkyle comprenant de 1 à 12 atomes de carbone ;
• L₁ et L₂ peuvent être identiques ou différents et représentent une chaîne hydrocarbonée linéaire ou ramifiée, saturée ou insaturée comprenant de 1 à 22 atomes de carbone ;
• B est une nucléobase éventuellement substituée, choisie dans le groupe constitué par les nucléobases puriques, les nucléobases pyrimidiques et les nucléobases hétérocycliques monocycliques ou bicycliques non naturelles dans lesquelles chaque cycle comprend de 4 à 7 atomes.

2. Oligonucléotide modifié selon la revendication 1, dans lequel ledit oligonucléotide est constitué d'une séquence d'ADN de 19 nucléotides.

3. Oligonucléotide modifié selon l'une quelconque des revendications 1 à 2, dans lequel ledit oligonucléotide est constitué de la séquence 5'-TTAGTTGGGGTTCAGTTGG-3' (SEQ ID NO : 1).

4. G-quadruplex parallèle tétramoléculaire comprenant 4 oligonucléotides modifiés identiques tels que définis dans l'une quelconque des revendications 1 à 3, dans lequel chacun des 4 résidus de guanine consécutifs compris au milieu de la séquence d'acide nucléique de chaque oligonucléotide forme respectivement des G-quartets avec les résidus de guanine correspondants des 3 autres oligonucléotides, lesdits G-quartets étant stabilisés par un piquetage π-π et une liaison hydrogène de Hoogsteen.

5. G-quadruplex parallèle tétramoléculaire selon la revendication 4, dans lequel ledit G-quadruplex comprend en outre un cation divalent qui coordonne lesdits G-quartets.

6. G-quadruplex parallèle tétramoléculaire selon la revendication 5, dans lequel ledit cation divalent est un cation Mg²⁺.

7. Composition comprenant des G-quadruplex parallèles tétramoléculaires selon l'une quelconque des revendications 4 à 6, dans laquelle les G-quadruplex parallèles tétramoléculaires se sont auto-assemblés en micelles.

8. Composition selon la revendication 7 comprenant en outre un principe actif hydrophobe hébergé dans lesdites micelles.

9. Utilisation de la composition selon la revendication 7 ou 8 comme véhicule.

10. Composition selon la revendication 7 ou 8 pour une utilisation comme médicament.

11. Utilisation d'un G-quadruplex parallèle tétramoléculaire selon l'une quelconque des revendications 4 à 6 dans la fabrication de dispositifs de nanotechnologie.

12. G-quadruplex parallèle tétramoléculaire selon l'une quelconque des revendications 4 à 6 pour une utilisation comme implant artificiel.
